# EUROPÄISCHE PATENTSCHRIFT

(11) **EP 2 538 950 B1**
(45) Veröffentlichungstag und Bekanntmachung des Hinweises auf die Patenterteilung: **31.07.2019**
(21) Anmeldenummer: 11703678.0
(22) Anmeldetag: 15.02.2011
(51) Int. Cl.: A61K 33/08, A61K 45/06, A61K 9/00, A61K 9/14

(54) **ZEOLITH-HALTIGE ZUBEREITUNGEN ENTHALTEND CLINOPTILOLITH UND DEREN ANWENDUNGEN**
ZEOLITE-CONTAINING PREPARATIONS CONTAINING CLINOPTILOLITE AND USES THEREOF
PRÉPARATIONS À BASE DE ZÉOLITE CONTENANT DE LA CLINOPTILOLITE ET LEURS APPLICATIONS

(30) Priorität: 22.02.2010 DE 102010008850
(43) Veröffentlichungstag der Anmeldung: 02.01.2013
(73) Patentinhaber: Biotake GmbH, 61479 Glashütten (DE)
(72) Erfinder: NICKCHEN, Reinhard, 61479 Glashütten (DE)
(74) Vertreter: Jacobi, Markus Alexander
(86) Internationale Anmeldenummer: PCT/EP2011/052201
(87) Internationale Veröffentlichungsnummer: WO 2011/101340

(56) Entgegenhaltungen:
- EP-A1- 1 316 530
- WO-A1-96/34828
- WO-A1-2009/133413
- WO-A1-2010/061355
- WO-A2-2010/140034
- CN-A- 101 007 261
- DE-U1-202010 013 541
- US-A1- 2005 031 708
- US-B1- 6 284 232
- ZEO HEALTH LTD: "SUPPLEMENTAL FACTS", INTERNET CITATION, 9. August 2003 (2003-08-09), XP002411120, Gefunden im Internet: URL:http://www.preventhangovers.zeohealth. com [gefunden am 2006-12-11]

## Beschreibung

Die vorliegende Erfindung betrifft Zubereitungen, die spezielle Aluminosilikate enthalten und die beispielsweise im Nahrungsmittelbereich oder als Arzneimittel verwendet werden können. Zahlreiche Aluminosilikate, und insbesondere Zeolithe, haben in den letzten Jahren verschiedene Anwendungen als Zusatzstoffe gefunden. Von Zeolithen ist bekannt, dass sie aufgrund ihrer besonderen Oberflächeneigenschaften beispielsweise in kosmetischen Zusammensetzungen oder als Nahrungsmittel-Zusatz verwendet werden können.

Seit Jahrhunderten ist bekannt, dass der Konsum von Alkohol, beispielsweise in Form von Wein und Bier, zu einem Unwohlsein und zu Beeinträchtigungen der körperlichen und geistigen Leistungsfähigkeit bei Mensch und Tier führen kann. Sowohl bei normalem Verbrauch wie auch bei leichten und schwereren Alkoholvergiftungen besteht ein Interesse daran, die vom Alkohol (insbesondere Ethanol) und seinen Abbauprodukten ausgelösten Beeinträchtigungen abzumildern bzw. zu vermeiden. Die umgangssprachlich auch als "Kater" oder "hang-over" bezeichneten Nebenwirkungen des Konsums von Alkohol bestehen unter anderem aus Kopfschmerzen, Beeinträchtigungen des Magens, Müdigkeit und allgemeinem Unwohlsein. Auch werden häufig Magenschleimhautreizungen, Erbrechen und Appetitlosigkeit nach Alkohol-Konsum beobachtet. Für die nach dem Alkohol-Konsum auftretenden Kopfschmerzen sind unter anderem die Dehydratation des Körpers und der Entzug wichtiger Komponenten aus dem Blut verantwortlich. Darüber hinaus werden durch den Alkohol spezielle Proteine beeinträchtigt.

Es hat in den letzten Jahren nicht an Versuchen gefehlt, Methoden bereitzustellen, die die Folgen eines mäßigen oder übermäßigen Gebrauchs von Alkohol abschwächen. Diese Methoden sollen zum einen allgemein anwendbar sein, zu keiner gesundheitlichen Beeinträchtigung führen, kostengünstig und ohne großen Aufwand durchführbar sein und ohne aufwändige Apparaturen realisiert werden können.
Schon seit langem ist bekannt, dass durch den Konsum von Aktivkohle vor, während oder nach dem Trinken alkoholischer Getränke die Wirkungen des Alkohols reduziert werden können. Nachteilig an Aktivkohle und anderen Zubereitungen, die Kohlenstoff enthalten, ist jedoch, dass diese nicht angenehm zu applizieren sind und praktische Nachteile haben.

Aus US 2005/0031708 ist eine Zusammensetzung bekannt, die einen Zeolith enthält und die für die Behandlung verschiedener Zustände eingesetzt werden kann. Der Zeolith ist mit ungefähr 50 bis 95 Gew.- % enthalten. Optional sind ein oder mehrere andere Bestandteile, wie verschiedene B-Vitamine, Folsäure, Pantothensäure, Calcium, Vitamin A, Vitamin C, Vitamin D, Vitamin E, Vitamin K, Eisen, Zink, Magnesium, Natrium, Bor, Jod, Mangan, Selen, Kupfer, Chrom, Omega 3- oder Omega 6-Fettsäuren. Der eingesetzte Zeolith kann beispielsweise ein Clinoptilolith-Zeolith sein mit einer durchschnittlichen Partikelgröße von 1 bis 2000 µm. Die Zusammensetzungen können für das Behandeln von Alkoholkonsum, gastrointestinale Erkrankungen, Vergiftungserscheinungen, sowie gegen Grippe oder gewöhnliche Erkältungskrankheiten eingesetzt werden.

Aus US 2004/0253322 ist eine Zusammensetzung bekannt, die eine Kombination von Mineralien, wie Zeolithen, und Pflanzenextrakten enthält, zur Behandlung von Neuropathien bei Diabetes. Die eingesetzten natürlichen Clinoptilolithe werden in einem Mahlwerk fragmentiert. WO 2010/018418 beschreibt eine Zubereitung basierend auf gemahlenem Clinoptilolith als therapeutischer Wirkstoff zur Bereitstellung von bioverfügbarem Silizium. US 4,579,742 offenbart ein Gemisch, charakterisiert durch verzögerte Gasfreisetzung, in Puder-, granulierter oder komprimierter Form, sowie dessen Verwendung in wasserlöslichen Zusammensetzungen zur Zubereitung von Kohlensäurehaltigen Getränken.

Eine Aufgabe der vorliegenden Erfindung besteht darin, Zusammensetzungen bzw. Zubereitungen bereitzustellen, die universell einsetzbar sind, kostengünstig hergestellt werden können, transport- und lagerfähig sind, und die bereits in geringe Mengen, vorzugsweise oral appliziert, die unerwünschten Wirkungen des Alkohols abzuschwächen bzw. zu beseitigen in der Lage sind. Dabei sollen eine verbesserte Wirksamkeit und eine erhöhte Verträglichkeit der Zusammensetzungen erreicht werden. Die Zubereitung soll vorzugsweise gegen die Nebenwirkungen von Alkohol helfen und zugleich eine Substitution und/oder ein Ersatz von Ernährungs-Komponenten (wie Vitaminen) ermöglichen. Die Doppelwirkung der Zubereitungen, nämlich Reduktion des Alkohol-Spiegels vor Erreichung der Leber und aktive Kater-Prävention ist besonders vorteilhaft. Offenbart wird eine Zubereitung (Z), die 20 bis 99,99 Gew.-%, insbesondere 50 bis 99,99 Gew.-%, oftmals auch 70 bis 99,99 Gew.-% mindestens einer Zeolith-Komponente, insbesondere einer Clinoptilolith-Komponente, enthält. Die Summe der Gewichtsprozent-Angaben soll zusammen mit den weiteren Komponenten (z. B. Hilfsstoffe wie Carbonate, Hydrogencarbonate, Geschmacksstoffe, Formulierungs-Hilfsmittel, etc.) für die gesamte Zubereitung 100 Gew.-% ergeben.

Ferner wird offenbart, dass die Zubereitung (Z) einerseits aus 20 bis 99,99 Gew.-%, insbesondere 50 bis 99,99 Gew.-%, oftmals auch 70 bis 99,99 Gew.-% Clinoptilolith, sowie andererseits aus 80 bis 0,01 Gew.-%, insbesondere 50 bis 0,01 Gew.-%, oftmals 30 bis 0,01 Gew.-% mindestens einer weiteren Komponente (K) bestehen kann. Erfindungsgemäß enthält die Zeolith-haltige Zubereitung (Z), die 50 bis 99,99 Gew.-% mindestens einer Clinoptilolith-Komponente (CL) und 50 bis 0,01 Gew.-% mindestens einer weiteren Komponente (K), wobei die Clinoptilolith-Komponente (CL) durch die Formel (C-1):

(Ca, K₂, Na₂, Mg)₄Al₈Si₄₀O₉₆ x 24 H₂O

beschrieben wird,
wobei die Clinoptilolith-Komponente (CL) fein gemahlen ist und eine mittlere Teilchengröße (D-50) von kleiner als 0,01 mm aufweist, und
wobei eine der weiteren Komponenten (K) eine mit einer Schutzhülle umgebende Carbonat- und/oder Hydrogencarbonat-Komponente ist.

Häufig sind insgesamt 1 bis 50 Gew.-%, oftmals auch 10 bis 50 Gew.-%, mehrerer der weiteren Komponente (K) in der Zubereitung enthalten (oftmals mehr als 10 verschiedene weitere Komponenten) und entsprechend 50 bis 99 Gew.-%, insbesondere 50 bis 90 Gew.-% der Clinoptilolith-Komponente. Die Summe der Gewichtsprozent-Angaben soll dabei für die gesamte Zubereitung 100 % ergeben.

Bei diesen weiteren Komponenten (K) kann es sich zum Beispiel um übliche Hilfskomponenten (H) (wie Carbonate, Hydrogencarbonate, polymere Hilfsstoffe, etc.) handeln, es kann sich jedoch auch um spezielle Komponenten, wie beispielsweise einzelne Vitamine (V) oder auch Kombinationen von Vitaminen handeln. Diese Zubereitungen können bevorzugt zur Prävention, Behandlung und/oder Therapie von Alkohol-Nebenwirkungen eingesetzt zu werden. Die Zubereitungen können ggf. aufgrund der besonderen physikochemischen Wirkung auch als Medizinprodukte vertrieben werden. Die Zubereitungen können auch eine geringe Restfeuchte (kleiner 6 %) enthalten.

Als spezielle Zeolithe haben insbesondere die Clinoptilolite in den letzten Jahren Verwendung in pharmazeutischen Zusammensetzungen und in kosmetischen Zubereitungen gefunden. Unter Clinoptiloliten versteht man chemisch komplexe Silikathaltige Mineralien. Diese sind in der Regel in der Lage, Wasser und andere Stoffe zu speichern und beispielsweise beim Erhitzen wieder abzugeben. Aufgrund ihrer Struktur können Zeolithe gleichförmige Poren und/oder Kanäle aufweisen, in denen andere Stoffe absorbiert werden können, weshalb Zeolithe auch als Molekularsiebe Verwendung gefunden haben.

Das Adsorptionsvermögen von Stoffen, insbesondere auch von Zeolithen kann durch Vermahlungsprozesse verändert werden. Die Zeolithe unter der Bezeichnung Clinoptilolithe können durch folgende allgemeine Formel (C) beschrieben werden:

**(Mⁿ⁺)x/n[(AlO₂)ₓ(SiO₂)_{y}]**•**mH₂O**, (C)

wobei M insbesondere für ein oder mehrere Metalle wie beispielsweise Lithium, Natrium, Kalium, Magnesium, Calcium, Silber, Zink, Kupfer, Mangan und/oder Eisen und/oder auch für Wasserstoff stehen kann. M steht bevorzugt für mehrere Metalle aus der Gruppe Natrium, Kalium, Magnesium, Calcium Titan und Eisen.
Der Index n steht für die Ladungszahl und ist beispielsweise 1, 2 oder 3. Auch x und y sind in der Regel, unabhängig voneinander, ganze Zahlen von 1 bis 60. Das Verhältnis von Silizium zu Aluminium in den Clinoptilolithen beträgt häufig zwischen 3:1 und 6:1. Die Anzahl an Wassermoleküle (m) beträgt in der Regel zwischen 0 und 30.

Erfindungsgemäß wird ein Clinoptilolith durch folgende Formel (C-1) beschrieben:

(Ca, K₂, Na₂, Mg)₄Al₈Si₄₀O₉₆ x 24H₂O (C-1)

Ein bevorzugt eingesetzter Clinoptilolith kann beispielsweise durch folgende chemische Zusammensetzungs-Grenzen (in Gew.-%) beschrieben werden:

| | |
|---|---|
| SiO₂ | 60,0 - 75 % |
| MgO | 0 - 2 % |
| Al₂O₃ | 10 - 15 % |
| Na₂O | 0,1 - 2 % |
| CaO | 1,5 - 8 % |
| TiO₂ | 0 - 0,5 % |
| K₂O | 0 - 5 % |
| Fe₂O₃ | 0 - 3 % |
| Si/Al | 4 - 7, insbesondere 4 - 6. |

Eine bevorzugt eingesetzte Clinoptilolith-Komponente hat beispielsweise in etwa folgende chemische Zusammensetzung (in Gew.-%):

| | |
|---|---|
| SiO₂ | 69 % |
| Al₂O₃ | 12,5 % |
| CaO | 4,5 % |
| K₂O | 2,5 % |
| MgO | 1 % |
| Fe₂O₃ | 1,3 % |
| Na₂O | 0,9 % |
| CaCO₃ | 0,1 %. |

Verschiedene Clinoptilolith-Komponenten sind im Handel erhältlich. Von Zeolithen, wie Clinoptilolithen, ist bekannt, dass sie aufgrund ihrer Struktur die besondere Fähigkeit aufweisen, andere Stoffe zu binden. Die Zubereitungen können neben der Clinoptilolith-Komponente auch zusätzlich 0,1 bis 10 Gew.-%, insbesondere 5 bis 10 Gew.-% einer Calcit-Komponente (Calciumcarbonat) enthalten.

Verschiedene Verfahren zur starken Verkleinerung (Mahlung) von Partikeln von Zeolithen werden unter anderem beschrieben in EP-A 0 823 884 (Mikronisierung von Zeolithen auf Teilchengrößen D-50 unter 0,8 Mikrometer) und in EP-A 1 316 530 (Mikronisierung von Zeolithen auf Teilchengrößen unter 0,5 Mikrometer). Diese sehr kleinen Partikel (mit Teilchengrößen unter 1 Mikrometer) haben in der praktischen Anwendung, z. B. im Nahrungsmittelbereich jedoch deutliche Nachteile.

Eine Aufgabe der vorliegenden Erfindung ist es, eine oral verabreichbare Zubereitung bereitzustellen, die Clinoptilolith und einen oder mehrere Hilfsstoffe enthält, die geeignet ist, die unerwünschten Wirkungen bzw. Nebenwirkungen von Alkohol zu vermeiden bzw. zu vermindern.

Diese Aufgabe wird gelöst durch eine Zeolith-haltige Zubereitung (Z), die 20 bis 99,99 Gew.-% (insbesondere 50 bis 99,99 Gew.-%, oftmals 70 bis 99,99 Gew.-%) mindestens einer Clinoptilolith-Komponente (CL) und 80 bis 0,01 Gew.-% (insbesondere 50 bis 0,01 Gew.-%, oftmals auch 30 bis 0,01 Gew.-%) mindestens einer weiteren Komponente (K) enthält.

Erfindungsgemäß wird eine Zeolith-haltige Zubereitung (Z), die 50 bis 99,99 Gew.-% mindestens einer Clinoptilolith-Komponente (CL) und 50 bis 0,01 Gew.-% an weiteren (z. B. 2 bis 15 verschiedenen) Komponenten (K) enthält, wobei es sich bei diesen weiteren Komponente (K) insbesondere handelt um:
Hilfskomponenten (H) (wie Dextrin, Stärke, Süßstoffe, Zucker (wie Fructose), Geschmacksstoffe, Gummi arabicum, Emulgator, Trennmittel, Antioxidantien, Aromastoffen und/oder um feinverteilte Hilfsstoffe wie Carbonate bzw. Hydrogencarbonate) und/oder
Vitamin-Komponenten (V), insbesondere ein oder mehrere Vitamine der B-Gruppe und/oder Vitamin C.

Besonders aktiv hat sich eine Zeolith-haltige Zubereitung (Z) erwiesen, die aus 50 bis 99,99 Gew.-% einer Clinoptilolith-Komponente (CL) und aus 50 bis 0,01 Gew.-% an weiteren Komponenten (K) besteht, wobei eine dieser weiteren Komponente (K) ein Vitamin-B-Komponente ist. Dabei kommt als Vitamin-B-Komponente häufig auch eine Kombination von Vitamin B12 mit einem oder mehreren Vitaminen der Gruppe B1, B2, B3, B5, B6, B7 und B9 infrage. Oftmals werden auch Kombinationen mit Vitaminen B und C eingesetzt.

Besonders aktiv hat sich die erfindungsgemäße Zeolith-haltige Zubereitung (Z) erwiesen, die aus 50 bis 99,99 Gew.-% einer Clinoptilolith-Komponente (CL) und aus 50 bis 0,01 Gew.-% an weiteren Komponenten (K) besteht. Mindestens eine dieser weiteren Komponente (K) ist eine Vitamin-B-Komponente. Erfindungsgemäß enthält sie (für eine verbesserte Applikation) auch mindestens eine Carbonat- und/oder eine Hydrogencarbonat-Komponente sowie eine Säurekomponente (wie Citronensäure) (als Hilfskomponenten), beispielsweise insgesamt in einer Menge von 10 bis 49,99 Gew.%. Durch die Zusammenwirkung von Carbonat/Hydrogencarbonat und Säurekomponente wird eine schnelle und verbesserte Verteilung der Komponenten der Zubereitung im Lösungsmittel (z. B. Wasser) erreicht.

Als Vitamin-B-Komponente kommt häufig auch eine Kombination von Vitamin B12 mit einem oder mehreren Vitaminen der Gruppe B1, B2, B3, B5, B6, B7 und B9 infrage.

Vorzugsweise enthält die Zeolith-haltige Zubereitung (Z) (bzw. sie besteht aus) 50 bis 99,99 Gew.-%, häufig 50 bis 80 Gew.-%, an Clinoptilolith-Komponente, sowie eine Kombination aus folgenden weiteren Vitamin-Komponenten:
Vitamin C, Vitamin E, Vitamin A, Nicotinsäureamid, Calcium-Pantothenat, Vitamin B6, Vitamin B2, Vitamin B1, Vitamin B12, Folsäure, Biotin und Vitamin D.

Darüber hinaus enthält sie für eine verbesserte Applikation vorzugsweise mindestens eine Carbonat- bzw. Hydrogencarbonat-Komponente sowie eine Säurekomponente (wie Citronensäure), beispielsweise insgesamt in einer Menge von 10 bis 49,99 Gew.%.
Das Mengenverhältnis der Hilfskomponenten (Summe Carbonat, Hydrogencarbonat sowie Säurekomponente) zu Clinoptilith-Komponente ist oftmals von 1,5 zu 1 bis 1 zu 1,5.

Die Erfindung betrifft auch eine Zeolith-haltige Zubereitung (Z), die aus 50 bis 99,99 Gew.-% einer Clinoptilolith-Komponente (CL) und aus 50 bis 0,01 Gew.-% an weiteren Komponenten (K) besteht, wobei mindestens drei dieser weiteren Komponenten (K) Vitamin-B-Komponenten sind. Dabei kommen als Vitamin-B-Komponenten Vitamin B12 und Vitamine der Gruppe B1, B2, B3, B5, B6, B7 und B9 infrage.

Gegenstand der Erfindung ist auch eine Zeolith-haltige Zubereitung (Z), die aus 50 bis 99,99 Gew.-% einer Clinoptilolith-Komponente (CL) und aus 50 bis 0,01 Gew.-% an weiteren Komponenten (K) besteht, wobei eine dieser weiteren Komponente (K) das Vitamin B12 ist und eine der weiteren Komponenten ein Hydrogencarbonat (insbesondere Natriumhydrogencarbonat) ist.

Das Mengenverhältnis von Clinoptilolith-Komponente (CL) zu Vitamin B12 ist vorzugsweise in etwa so zu wählen, dass auf eine Menge von 10 g Clinoptilolith-Komponente (CL) etwa 0,001 mg bis 0,02 mg Vitamin B12 eingesetzt werden.

Die Vitamin-B-Komponente wird häufig auch in Kombination von Vitamin B12 mit drei oder mehreren Vitaminen der Gruppe B1, B2, B3, B5, B6, B7 und B9 eingesetzt. Die Gesamtmenge an Vitamin B-Komponenten beträgt dabei häufig - bezogen auf 10 g Clinoptilolith-Komponente (CL) - etwa 0,005 mg bis 20 mg Vitamin B.

Die Erfindung betrifft auch eine Zeolith-haltige Zubereitung (Z), welche aus 50 bis 99,99 Gew.-% (häufig auch 50 bis 80 Gew.-%), einer Clinoptilolith- Komponente (CL) besteht, wobei diese fein gemahlen ist. Dabei hat sich eine mittlere Teilchengröße (D-50) von kleiner als 0,02 mm, erfindungsgemäß von kleiner 0,01 mm als besonders geeignet erwiesen. Vorzugsweise weisen mindestens 85 % der Teilchen eine Partikelgröße von kleiner als 0,01 mm auf. Der Anteil an Zeolith-Partikeln mit einer mittleren Teilchengröße (D-50) von kleiner als 0,002 mm sollte dabei jedoch nicht mehr als 20 %, insbesondere nicht mehr als 10 % betragen. Der Anteil an Zeolith-Partikeln mit einer mittleren Teilchengröße (D-50)
von kleiner als 0,001 mm sollte dabei jedoch nicht mehr als 5 %, insbesondere nicht mehr als 2 % betragen.

Eine bewährte mittlere Teilchengrößen-Verteilung (Gew.-%) für die Zeolith-Partikel kann z. B. wie folgt aussehen:

| | |
|---|---|
| etwa 20 % der Partikel: | 0,01 bis 0,02 mm |
| etwa 60 bis 65 % der Partikel: | 0,002 bis 0,01 mm |
| höchstens 20, meistens etwa 15 % der Partikel: | kleiner als 0,002 mm. |
| höchstens 2, meistens weniger 1 % der Partikel: | kleiner als 0,001 mm. |

Die Zerkleinerung der Clinoptilolith- Komponente (und der weiteren Komponenten) kann nach bekannten Methoden erfolgen, beispielsweise durch Feinmahlung oder Feinstmahlung in entsprechenden Mühlen wie Mahlkörpermühlen (siehe Handbuch der Mechanischen Verfahrenstechnik (H. Schubert, Wiley-Verlag, 2003, S.299ff.). Die Bestimmung der Partikelgröße und der Partikelgrößenverteilung erfolgt nach dem Fachmann bekannten Verfahren (wie Sieben, Mikroskopie, etc.). Der Anteil an Zeolith-Partikeln mit einer mittleren Teilchengröße (D-50) im Bereich von 0,01 bis 0,001 mm sollte dabei vorzugsweise mindestens 70 %, oftmals mindestens 80 %, insbesondere mindestens 90 % betragen. Die weiteren Komponenten der Zusammensetzung (z. B. die Carbonat bzw. Hydrogencarbonat-Komponenten) können entsprechend gemahlen eingesetzt werden, dies ist jedoch nicht immer erforderlich.

Erfindungsgemäß ist eine Zeolith-haltige Zubereitung (Z), die aus 50 bis 99,99 Gew.-% einer Clinoptilolith-Komponente (CL) und aus 50 bis 0,01 Gew.-% an weiteren Komponenten (K) besteht, wobei eine dieser weiteren Komponente (K) ein Oberflächen-modifizierter Hilfsstoff (H) ist. Als Oberflächen-modifizierte Hilfsstoffe kommen erfindungsgemäß Komponenten infrage, die zur Regulierung des pH-Wertes eingesetzt werden (z. B. Carbonat bzw. Hydrogencarbonat-Komponente), und die erfindingsgemäß mit einer Schutzhülle umgeben werden, um einen negativen Einfluss auf die weiteren Komponenten zu vermeiden.

Beispielsweise kann als Hilfskomponente (H) eine CO₂-abgebende, anorganische Substanz, wie Soda (Natriumcarbonat) verwendet werden. Als Oberflächen-Modifizierung kann diese Hilfskomponente von einer Polymer-Schicht umgeben werden und/oder durch thermische Behandlung /Verbackung) an der Oberfläche "inaktiviert" werden. Von diesen "modifizierten" Hilfskomponenten werden beispielsweise 0,01 bis 49,99, insbesondere 0,1 bis 49,99 Gew.-% eingesetzt.

Die Oberflächen-Modifizierung kann sich auf die Clinoptilolith-Komponente (CL), auf die weiteren Komponenten (K) und/oder auf die Hilfsstoffe (H) beziehen. Eine wichtige Anforderungen an eine Oberflächen-Modifizierung ist, dass sie nicht klebrig ist, gut an der Substratoberfläche haftet und stabil ist gegenüber mechanischer Belastung. Zudem sollten sich die Polymere des Filmüberzugs schnell und einfach herstellen und aufbringen lassen.

Die Filmüberzugslösung sollte ferner eine geringe Viskosität bei ausreichend hohem Feststoffgehalt aufweisen, um eine gute Verarbeitung, z. B. in einem Sprühverfahren, zu gewährleisten. Zum Überziehen von Tabletten werden seit Jahren filmbildende Polymere eingesetzt, beispielsweise Cellulosederivate wie Hydroxypropylcellulose, Hydroxypropylmethylcellulose, Methacrylsäure-Copolymere oder auch Polyvinylalkohol-Copolymere. Auch der Einsatz von Polymeren wie Polyethylenglykol und Polyvinylpyrrolidon als filmbildende Komponenten in Beschichtungszusammensetzungen ist bekannt. Der in GB 1 021 178 beschriebene Filmüberzug dient in erster Linie dem Schutz der darunter liegenden Wachsbeschichtung und soll deren Lagerstabilität erhöhen. US 4,060,598 beschreibt ein Verfahren zur Herstellung von beschichteten, wirkstoffhaltigen Trägern, wobei die Beschichtung durch das Aufbringen einer wässrigen Zusammensetzung mit einer wasserunlöslichen und einer wasserlöslichen Komponente durchgeführt wird. In WO 2006/102446 wird eine mehrfach beschichtete pharmazeutische Zubereitung mit modifiziertem Auflöseverhalten beschrieben.

Die erfindungsgemäße Oberflächen-Modifizierung kann z. B. aus einer Zusammensetzung mit Polyethylenglykol und einem Polyvinylpyrrolidon sowie optional weiteren, nicht polymeren Hilfsmitteln bestehen. Weiterhin soll die Erfindung ein verbessertes Verfahren zur Herstellung einer beschichteten Zusammensetzung bereitstellen. Die erfindungsgemäßen Überzüge zeichnen sich neben hervorragendem Glätte durch niedrige Klebrigkeiten und Lagerstabilität aus.Die Erfindung betrifft auch eine Zeolith-haltige Zubereitung (Z), wobei es sich um eine orale Zubereitung in Pulverform handelt, die eine Clinoptilolith-Komponente (CL) sowie mehrere Vitamin-B-Komponenten enthält.

Die Pulver-förmigen Zubereitungen können beispielsweise in Aufreißbeuteln (beispielsweise aus Aluminiumsfolie oder Kunststofffolie) vor dem Einfluss von Feuchtigkeit, Licht und Temperatur geschützt werden. Eine typische Dosierung der erfindungsgemäßen Zubereitung beträgt 5-20 g (oder auch 5-15 g) pro Verpackungseinheit.

Gegenstand der vorliegenden Erfindung ist aber auch ein Verfahren zur Herstellung einer Zeolith-haltigen Zubereitung (Z), bei dem zunächst die Clinoptilolith-Komponente (CL) durch ein Mahlverfahren auf eine mittlere Teilchengröße (D-50) von kleiner als 0,02 mm, häufig auch kleiner 0,01 mm gebracht wird, und anschließend die Clinoptilolith-Komponente (CL) mit der oder den weiteren Komponenten (K) vermischt wird. Typische Mahlverfahren zur Erzeugung dieser Teilchengröße sind dem Fachmann geläufig. Neben der Komponente (CL) können auch die weiteren Komponenten (z.B. die anorganischen Hilfsstoffe) einem Vorgang der Teilchengrößen- Verminderung unterzogen werden.

Das Verfahren zur Herstellung der Zeolith-haltigen Zubereitung (Z) kann auch den weiteren Schritt der Oberflächen-Modifizierung der Clinoptilolith-Komponente (CL) oder der weiteren Komponenten beinhalten, wobei eine entsprechende Schicht aufgebracht wird.

Die Erfindung betrifft auch die Verwendung der erfindungsgemäßen Zeolith-haltigen Zubereitung (Z) zur Prävention oder Behandlung der Nebenwirkungen von Alkohol. Auch die Verwendung der speziellen Kombination aus einer Clinoptilolith-Komponente (CL) und mindestens einer Vitamin B12-Komponente zur Prävention oder Behandlung der Nebenwirkungen von Alkohol ist Gegenstand dieser Erfindung.

Unter Schutz gestellt wird ebenfalls eine Dosier-Einheit, wie beispielsweise ein Aufreißbeutel, enthaltend 5-20 g, vorzugsweise 6-15 Gramm einer pulverförmigen, Zeolith-haltigen Zubereitung (Z). Beim Einsatz dieser Aufreißbeutel wird der Inhalt entweder direkt oral appliziert oder er wird in ein Gefäß mit einem flüssigen Medium vermischt und dann durch Trinken aufgenommen. Beispielsweise kann das Pulver in Wasser (z. B. 0,05 bis 0,3 Liter) eingebracht und dann oral verabreicht werden.

Neben der Zeolith-Komponente und den Hilfsstoffen (H) enthält die Zubereitung in einer bevorzugten Ausführungsform auch ein oder mehrere Vitamine (V).
Bei umfangreichen Untersuchungen hat sich gezeigt, dass insbesondere eine Zubereitung mit einer Kombination von Clinoptilolith (CL) mit einzelnen oder mehreren Vitaminen der B-Gruppe besondere Vorteile bei der Anwendung mit sich bringt.

Der Gruppe der B-Vitamine gehören zahlreiche Substanzen an, die in pflanzlichen und tierischen Lebensmitteln vorkommen. Besonders zu nennen sind an dieser Stelle das Vitamin B1 (Thiamin), das Vitamin B2 (Riboflavin), das Vitamin B3 (Nicotinsäure), das Vitamin B5 (Pantothensäure), das Vitamin B6 (Pyridoxin), das Vitamin B7 (Biotin), das Vitamin B9 (Folsäure), sowie Vitamin B12 (Cobalamin). Die erfindungsgemäße Zubereitung kann neben der Hauptkomponente Clinoptilolithe auch mehrere (zum Beispiel 2, 3, 4, 5 oder 6) verschiedene Vitamine der B-Gruppe enthalten.

Die Erfindung bezieht sich insbesondere auf eine orale Zubereitung, die neben einem oder mehreren Clinoptiolith-Komponenten auch das Vitamin B12 (Cobalamin) enthält.

Wenngleich dieses Vitamin B12 in zahlreichen Nahrungsmitteln natürlich vorkommt und auch im menschlichen Dickdarm synthetisiert werden kann, kommt Vitamin B12 als Nahrungsergänzungsmittel besondere Aufmerksamkeit zu. Es hat sich bei Untersuchungen überraschend gezeigt, dass eine Zubereitung, die Clinoptilolith und Vitamin B12 (und gegebenenfalls weitere Komponenten) enthält, besonders gut geeignet ist, die Nebenwirkungen und/oder Nachwirkungen von Alkohol abzuschwächen oder aufzuheben.

Die erfindungsgemäße Zubereitung wird im Allgemeinen oral verabreicht. Geeignete feste oder flüssige galenische Zubereitungsformen sind insbesondere Pulver-Zubereitungen aber auch Tabletten, Granulate, Dragees, Kapseln, Mikrokapseln, Sirupe, Emulsionen und Suspensionen sowie Zubereitungen mit protrahierter Wirkung.

Bei der Herstellung der Zubereitung kommen ggf. physiologisch geeignete Hilfsmittel (H) wie Träger-, Binde-, Überzugs-, Quellungs-, Schmiermittel sowie Geschmacksstoffe (z.B. Zitronensäure, Orangenaroma), Süßungsmittel (Zucker, Süßstoffe) oder Lösungsvermittler zum Einsatz. Als häufig verwendete Hilfsstoffe (H) werden beispielsweise Natriumcarbonat und Natriumhydrogencarbonat, Magnesiumcarbonat, Titanoxid, Lactose, Mannit, Fructose, andere Zucker, Milcheiweiß, Gelatine, Stärke, Zellulose und ihre Derivate, tierische und pflanzliche Öle, Polyethylenglykole, sowie Lösungsmittel wie Wasser genannt.

Vorzugsweise werden die Zubereitungen in Dosiereinheiten (beispielsweise in Form von Aufreiß-Beuteln) hergestellt und verabreicht, wobei jede Einheit als "aktiven" Bestandteil eine bestimmte Dosis von Clinoptilolith (bzw. Clinoptilolith und Vitamin B12) enthält. Bei festen Dosiereinheiten kann diese Menge an Clinoptilolith (bzw. Clinoptilolith und Vitamin B12) beispielsweise 1 g bis 30 g, bevorzugt 2 g bis 20 g, insbesondere 4 g bis 15 g betragen. Die Dosierung von etwa 8 g Clinoptilolith pro Einheit (z. B. Aufreiß-Beutel) hat sich besonders bewährt. Häufig sind etwa 2 bis 7 g an Carbonat- und/oder Hydrogencarbonat (auch Carbonat-Komponente genannt) enthalten. In einer typischen Formulierung enthält die Zubereitung auf 8 g Clinoptilolith etwa 50-200 mg an weiteren Komponenten (außer Carbonat, Hydrogencarbonat und Säurekomponente). Die Zubereitung enthält oftmals auch auf 8 g Clinoptilolith etwa 2 bis 7 g Carbonat-Komponente (z. B. 2g Hydrogencarbonat und 0,3 g Carbonat) sowie 2 bis 5 g Citronensäure (als Säure-Komponente.

Die optimal zu verabreichende Gesamtdosis an Clinoptilolith (bzw. Clinoptilolith und Vitaminen wie B12) kann abhängig sein vom Körpergewicht, Geschlecht, Alter und dem allgemeinen Zustand der Person. Die einzusetzende Menge hängt nur natürlich auch von der Menge des konsumierten oder zu konsumierenden Alkohols ab.

Die Verabreichung der Dosis kann zwar als Einmalgabe in Form einer einzelnen Dosiereinheit erfolgen, es kann sich jedoch als vorteilhaft erweisen, kleinere Dosiereinheiten durch Mehrfachgabe zum Einsatz zu bringen, beispielsweise durch Verabreichung einer Zubereitung vor dem Alkoholkonsum und einer weiteren Verabreichung der Zubereitung nach dem Alkoholkonsum. Besonders vorteilhaft hat sich beim Einsatz der oralen Zubereitungen die Vergabe einer Dosis von 2 g bis 20 g, insbesondere 4 g bis 15 g an Clinoptilolith im Abstand von 0,5 bis 2 Stunden erwiesen. Durch dieses Applikationsschema kann beispielsweise ein beschwerdefreier Nachtschlaf (auch ohne Unterbrechungen der Nachtruhe) erreicht werden.

Die Erfindung betrifft auch die Verwendung von Clinoptilolith (bzw. einer Kombination von Clinoptilolith und Vitamin B12) zur Behandlung und/oder Prävention der Begleiterscheinungen eines Alkoholkonsums. Die Erfindung betrifft auch die Verwendung zur Herstellung einer Zubereitung beziehungsweise eines Arzneimittels zur Behandlung und/oder Prävention von Kopfschmerzen und verwandter Nebenwirkung-Erscheinungen im Zusammenhang mit einem Alkohol-Konsum.

Die erfindungsgemäßen Zubereitungen werden in der Regel dadurch hergestellt, dass man Clinoptilolith (bzw. eine Kombination von Clinoptilolith und Vitamin B12) mit einem oder mehreren physiologisch geeigneten Hilfsstoffen in eine geeignete Darreichungsform bringt. Dies kann beispielsweise durch einfaches Vermischen der einzelnen Komponenten erfolgen.

Die in pulverförmigen Zubereitungen eingesetzten Teilchen (beispielsweise der Hilfsstoffe) können auch oberflächenmodifiziert sein. Die Oberflächenmodifizierung von Teilchen mit bestimmten Überzügen (polymere Schutzschichten) oder mit Gruppen, um die Teilchen mit einer oder mehreren zusätzlichen Funktionen zu versehen, ist dem Fachmann vertraut. Er kann solche oberflächenmodifizierten Teilchen ohne weiteres herstellen oder gegebenenfalls im Handel erwerben. Oberflächenmodifizierte Teilchen werden im allgemeinen durch Umsetzung der Teilchen mit geeigneten Oberflächen-Modifizierungsmitteln erhalten, wobei die Zugabe des Oberflächen-Modifizierungsmittel auch in situ während der Herstellung der Teilchen erfolgen kann. Die Umsetzung erfolgt unter solchen Bedingungen, dass eine Anbindung des Modifizierungsmittels, z. B. durch chemische Bindung oder durch Wechselwirkung, auf der Oberfläche der Teilchen erfolgt. Die Bedingungen der Oberflächenmodifizierung bzw. -Beschichtung hängen naturgemäß von der Art der Teilchen (z. B. anorganische Hilfskomponente wie Hydrogencarbonat) und der Oberflächenmodifizierungsmittel ab.

Es kann ein einfaches Rühren bei Raumtemperatur ausreichen, gegebenenfalls ist aber auch ein Energieeintrag, z.B. durch Erwärmen notwendig. Der Belegungsgrad der Teilchenoberflächen der Partikel mit den Modifizierungsmitteln kann z.B. durch das eingesetzte Mengenverhältnis der Edukte gesteuert werden. So hat es sich als besonders vorteilhaft erwiesen, wenn einzelne Komponenten der erfindungsgemäßen Zubereitung zur Vermeidung von Wechselwirkungen mit anderen Komponenten mit einer Schutzschicht (z. B. aus Wasserlöslichem Polymer) überzogen werden.

Zur Vermeidung beziehungsweise Verminderung der Nebenwirkungen von Alkohol kann Clinoptilolith (bzw. eine Kombination von Clinoptilolith und Vitamin B12) selbst als Substanz (bzw. Gemisch) verwendet werden, vorzugsweise liegt die Clinoptilolith-Komponente jedoch mit einem weiteren Hilfsstoff (H) in Form einer Zusammensetzung vor.

Der Hilfsstoff muss natürlich verträglich sein, in dem Sinne, dass er mit den anderen Bestandteilen der Zubereitung kompatibel ist und nicht gesundheitsschädlich für den Anwender ist. Der Träger kann ein Feststoff oder eine Flüssigkeit oder auch beides sein und wird vorzugsweise mit der Clinoptilolith-Komponente als Einzeldosis formuliert, beispielsweise als auflösbares (bzw. suspendierbares) Pulver, das von 30 % bis 99,99 Gew.-% der Clinoptilolith-Komponente enthalten kann. Weitere "aktive Substanzen", wie z. B. Vitamine (V) können ebenfalls vorhanden sein, einschließlich des besonders bevorzugten Vitamins B12.

Die erfindungsgemäßen Zubereitungen können nach einer der bekannten Methoden hergestellt werden, die im Wesentlichen darin bestehen, dass die Clinoptilolith-Komponente ggf. mit verträglichen Hilfsstoffen (H) gemischt werden. Bei der Herstellung der erfindungsgemäßen Zubereitungen (Z) hat sich gezeigt, dass eine Verkleinerung der Partikelgröße, insbesondere ein Vermahlen der Clinoptilolith-Komponente besondere Vorteile mit sich bringt. Dabei sollten zu grobe Teilchen (größer 20 Mü) ebenso wie zu kleine Teilchen (kleiner 1 Mü) möglichst selten auftreten.

Im Allgemeinen werden die Zubereitungen durch gleichmäßiges und homogenes Vermischen der (ggf. auf eine gewünschte Korngröße gemahlenen) Clinoptilolith-Komponente (gegebenenfalls mit Überzug versehen) mit weiteren Komponenten, also Vitamin-Komponenten und Hilfskomponenten (wie Dextrin, Stärke, Süßstoffe, Zucker, Gummi arabicum, Emulgator, Trennmittel, Antioxidantien, Aromastoffen, Citronensäure) sowie mit feinverteiltem Hilfsstoff (z.B. Carbonat bzw. Hydrogencarbonat, ggf. mit Überzug versehen) hergestellt, wonach das Produkt, falls erforderlich, portioniert und abgefüllt wird. Es kann auch eine Tablette hergestellt werden, indem ein Pulver verpresst oder geformt wird, gegebenenfalls mit einem oder mehreren zusätzlichen Bestandteilen.

Gepresste Tabletten können durch Tablettieren der Komponenten in frei fließender Form, wie beispielsweise einem Pulver, gegebenenfalls gemischt mit einem Bindemittel, Gleitmittel, inertem Verdünner und/oder einem (mehreren) oberflächenaktiven/ dispergierenden Mittel in einer geeigneten Maschine hergestellt werden.

Geformte Tabletten können durch Formen des pulverförmigen, ggf. mit einem inerten flüssigen Verdünnungsmittel befeuchteten Clinoptilolith in einer geeigneten Maschine hergestellt werden. Zubereitungen, die für eine orale (z. B. sublinguale) Verabreichung geeignet sind, umfassen auch Lutschtabletten, die zum Beispiel einen Geschmacksstoff enthalten, und Pastillen. Eine typische erfindungsgemäße Zubereitung enthält in etwa:

| | |
|---|---|
| Clinoptilolith | 8 bis 10 Gewichtsteile (fein gemahlen) |
| Vitamine C, D, E, A, B | 0,1 bis 0,5 Gewichtsteile |
| Carbonat-Komponenten | 2 bis 10 Gewichtsteile (fein gemahlen) |
| Citronensäure | 2 bis 5 Gewichtsteile sowie ggf. |
| Polymer-Hilfskomponenten | 0 bis 2 Gewichtsteile. |

Die vorliegende Erfindung betrifft auch die Herstellung einer (pharmazeutischen) Zubereitung enthaltend Clinoptilolith (bzw. eine Kombination von Clinoptilolith und Vitamin B12) zur Prävention und Behandlung von Alkohol-Vergiftungen (Intoxikationen), sowie der Nebenwirkungen, die auf einem Alkohol-Übermaß basieren. Hierzu gehören u. a. starke Kopfschmerzen, Krämpfe, Schlafstörungen, Erbrechen, Sprachstörungen und Panikattacken. Die Erfindung betrifft auch die Verwendung der Zubereitung zur Prävention, Verminderung und Behandlung von Alkohol-Wirkungen bzw. Nebenwirkungen, insbesondere wenn sie im Zeitraum von 1 Stunde vor bis 1 Stunde nach Alkoholaufnahme appliziert wird.

Die Erfindung betrifft auch ein Verfahren zur Herstellung einer Zubereitung enthaltend Clinoptilolith und einen oder mehrere Hilfsstoffe (H), bei dem Clinoptilolith zunächst einer Zerkleinerung auf eine mittlere Korngröße (D-50) von kleiner als 20 µm, insbesondere kleiner 10 µm unterzogen wird und anschließend mit den Hilfsstoffen (H) bzw. weiteren Komponenten (K) vermischt wird. Das erfindungsgemässe Verfahren zur Herstellung der Zubereitung weist beispielsweise die folgenden Schritte auf:
a) Zerkleinern eines Clinoptilolith durch Scherung und/oder Prallung zu Partikeln mit einer mittleren Korngröße (D-50) von kleiner als 20 µm, insbesondere kleiner 10 µm;
b) Vermischen der Clinoptilolith-Partikel mit den anderen Komponenten; und
c) Portionieren und Abfüllen der Mischung.

Der Anteil an Clinoptilith-Partikel mit einer mittleren Teilchengröße (D-50) im Bereich von 0,01 bis 0,002 mm kann vorzugsweise auf etwa 80 %, insbesondere etwa 90 %, eingestellt werden. Die weiteren Komponenten der Zusammensetzung können entsprechend gemahlen werden, dies ist jedoch nicht immer erforderlich.

Durch die Anwesenheit einer Oberflächen-Modifizierung bei Hilfsstoffen (H), wie weiter oben beschriebenen, kann, soweit dies erforderlich sein sollte, beim Portionieren und bei der Lagerung eine Agglomeration der Partikel verhindert werden, insbesondere bei Pulver-Zubereitungen können die CO₂-spendenden Hilfsstoffe durch thermische Behandlung (>500°C) an der Oberfläche calciniert werden.

Die Schritte a) können auch durch Nassvermahlung in einer Mühle mit Mahlhilfskörpern durchgeführt werden. Besonders vorteilhaftkann es sein, wenn die Schritte a) und b) in einer Rührwerkskugelmühle erfolgen. Alternativ können die Schritte a) und b) auch in einem Dreiwalzwerk durchgeführt werden. Vorzugsweise werden die in Schritt a) erzeugten Partikel auf mittlere Korngrößen kleiner 20 µm, insbesondere kleiner 10 µm und bevorzugt auf Größen zwischen 2 µm und 15 µm vermahlen. Gewöhnlich erfolgt die Aufbereitung der anorganischen Partikel nach deren Herstellung aus dem Grobgut durch Zerkleinerung, wie Brechen und stufenweisem Mahlen mit abschließendem Feinmahlen auf die gewünschte Korngröße. Häufig werden dabei auch war Kugelmühlen verwendet, mit denen eine vergleichsweise exakte durchschnittliche Korngröße eingestellt werden kann. Der dabei angewandte Trockenmahlprozess kann dazu führen, dass der Energieeintrag auch zur unspezifischen statistischen Spaltung der Partikel führt.

Die Erfindung betrifft auch die anorganischen, gemahlenen Zeolith-Partikel, die einen mittleren Durchmesser (D-50) von 2 bis 20 µm, insbesondere 2 bis 15 µm aufweisen. Die Bestimmung der Partikelgröße und der Größenverteilung erfolgt nach dem Fachmann bekannten Methoden. Beispielsweise ist eine mikroskopische Untersuchung möglich.

Die Erfindung wird durch die nachfolgenden Beispiele näher erläutert:

### Beispiel 1 (Ausgangskomponente für die Herstellung der Zubereitung)

Die für die Herstellung der erfindungsgemäßen Zubereitung eingesetzte Clinoptilolith-Komponente wurde sowohl in ungemahlenem Zustand, in gemahlenem Zustand (mit einer mittleren Teilchengröße von etwa 0,1 mm) und in besonders fein gemahlenem Zustand (mit einer mittleren Teilchengröße von kleiner als 0,01 mm) eingesetzt.
a) Materialbezeichnung für die Zeolith-Komponente:

| | |
|---|---|
| Materialsname | Naturzeolith |
| chemische Benennung | hydratisiertes Alkali-Erdalkali-Alumosilicat |
| Mineralform | Clinoptilolith |
| Struktur | Tektosilicat |
| empirische Formel | (Ca, K₂, Na₂, Mg) Al₈Si₄₀O₉₆ x 24H₂O |

b) Chemische Zusammensetzung eines ersten eingesetzten Zeolithen (Clinoptilolith):

| | |
|---|---|
| SiO₂ | 65,0 - 71,3 % |
| MgO | 0,6 - 1,2 % |
| Al₂O₃ | 11,5 - 13,1 % |
| Na₂O | 0,2 - 1,3% |
| CaO | 2,7 - 5,2 % |
| TiO₂ | 0,1 - 0,3 % |
| K₂O | 2,2 - 3,4 % |
| Fe₂O₃ | 0,7 - 1,9 % |
| Si/Al | 4,8 - 5,4 |

c) Physikalische und chemische Eigenschaften des Zeolithen:

| | |
|---|---|
| Erweichungstemperatur | 1.260°C |
| Schmelztemperatur | 1.340°C |
| Schüttgewicht | nach Körnung |
| Fließtemperatur | 1.420°C |
| Druckfestigkeit | 33 Mpa |
| spezifisches Gewicht | 2.200 - 2.400 kg/m³ |
| Porosität | 24-32% |
| Wasseraufnahme | 34- 36% |
| pH-Wert (in Wasser) | 6,6 - 7,2 |
| Härte nach Mohs | 1,5 - 2,5 |
| Eff. Porendurchmesser | 4 nm |
| Dichte | 70% |
| Weißgrad | 70% |

Eine weitere, eingesetzte Clinoptilolith-Komponente hat beispielsweise in etwa folgende chemische Zusammensetzung (in Gew.-%):

| | |
|---|---|
| SiO₂ | 69 % |
| Al₂O₃ | 12,5 % |
| CaO | 4,5 % |
| K₂O | 2,5 % |
| MgO | 1 % |
| Fe₂O₃ | 1,3 % |
| Na₂O | 0,9 % |
| CaCO₃ | 0,1 %. |

d) Komponenten der untersuchten Zubereitung (pro Dosiereinheit):

| | |
|---|---|
| Clinoptilolith | 8,0 g |
| Vitamin C | 120 mg |
| Vitamin E | 10 mg |
| Vitamin A | 0,8 mg |
| Nicotinsäureamid | 36 mg |
| Calcium-Pantothenat | 12 mg |
| Vitamin B6 | 4 mg |
| Vitamin B2 | 3,2 mg |
| Vitamin B1 | 2,8 mg |
| Vitamin B12 | 3 µg |
| Folsäure (V-B9) | 0,4 mg |
| Biotin (V-B7) | 0,3 mg |
| Vitamin D | 5 µg |
| Weitere Komponenten | ad 15 g |

Als weitere Komponenten werden z. B. 2 g Natriumhydrogencarbonat, 0,3 g Natriumcarbonat, 0,4 g Fructose, etwa 4 g Citronensäure sowie kleiner 0,1 g Farbstoffe eingesetzt.

Der Anteil an Clinoptilith-Partikel mit einer mittleren Teilchengröße (D-50) im Bereich von 0,015 bis 0,002 mm beträgt größer 80 %. Der Anteil an Partikel mit einer mittleren Teilchengröße (D-50) von kleiner als 0,001 mm beträgt weniger als 2 %.

Die weiteren Komponenten der Zusammensetzung werden zwar pulverförmig, jedoch nicht entsprechend fein gemahlen eingesetzt.

Diese Zusammensetzung kann durch Vermischen der Komponenten kostengünstig hergestellt und als Pulver portioniert werden (z. B. in der genannten Menge pro Aufreißbeutel). Sie ist über viele Wochen und Monate lagerstabil.

### Beispiel 2 (Beeinflussung des Atemalkohol-/Blutalkoholspiegels durch die Zubereitung gemäß Beispiel 1)

Für die nachfolgende Untersuchung wurde eine Zubereitung eingesetzt, in der die Hauptkomponente der Zusammensetzung (Clinoptilolith-Komponente) eine mittlere Teilchengröße von kleiner als 0,01 mm aufwies. Besonders geeignet erwies sich eine Zerkleinerung der Partikel, bei der mindestens 85 % der Teilchen eine Teilchengröße von weniger als 0,01 mm aufweisen. Auch kann es von Vorteil sein, wenn die Teilchengrößenverteilung eng ist.

Als Teilnehmer der Untersuchung fungierten 19 gesunde (weibliche (9) und männliche (10)) Probanden im Alter zwischen 20 und 39 Jahren. Der Body Mass Index (BMI) der Teilnehmer rangierte bei 17 dieser Personen zwischen 19,1 und 23,9 (normalgewichtige Probanden), zwei männliche Probanden hatten einen BMI von 27,1 und 26, 2.

Es erfolgte zunächst in einem festgelegten Zeitintervall (120-173 Minuten) eine Aufnahme von alkoholischen Getränken, zuvor erfolgte eine Atemalkoholbestimmung (nach üblichen Methoden) als "Leerwert". Hierbei waren alle 19 Probanden mit 0,00 Promille nüchtern. Die Trinkmenge sowie die Getränkeauswahl erfolgten individuell, zur Verfügung standen folgende Alkoholika: Bier, Wein, Drinks auf Basis von Gin oder Wodka. Zum Teil hatten die Teilnehmer vor der "Trinkphase" ein leichtes Abendessen zu sich genommen.
Während der Trinkphase wurden z.T. Speisen in Form von Salz- oder Süßgebäck aufgenommen, weiterhin wurden individuell verschiedene nichtalkoholische Getränke wie Wasser zusätzlich getrunken. Zu festgelegten Zeitpunkten erfolgte dann ein Trinkstopp, alkoholische Getränke durften dann nicht mehr aufgenommen werden. 20 bis 30 Minuten nach der letzten Alkoholaufnahme erfolgte eine weitere Messung der Atemalkoholkonzentration (AAK nach Trinkende = AAK 0'). Unmittelbar im Anschluss erfolgte die Aufnahme der Zubereitung gemäß Beispiel 1 (15g Pulver-Zubereitung aus Beispiel 1, gelöst in ca. 200ml Wasser).

In zeitlichen Intervallen von 30, 60 und 90 Minuten wurde jeweils die Atemalkoholkonzentration der Probanden bestimmt und protokolliert. Weiterhin wurden die subjektiven Alkoholauswirkungen (Sprachfähigkeit, Auf-einer-Linie-Gehen, etc.) bzw. die Änderungen nach Einnahme der Zubereitung erhoben. Unverträglichkeiten oder medizinisch erkennbare Nebenwirkungen konnten zu keiner Zeitbeobachtet werden. Es wurde Folgendes beobachtet:
- Die Alkoholreduktion bei 16 Probanden betrug zwischen 45% und 61% nach 30 Minuten nach Verabreichung der Zubereitung, bei 3 Probanden zwischen 5% und 20%
- eine weitere Reduktion des Alkoholgehalts um ca. 10% nach 60 Minuten nach Verabreichung der Zubereitung
- durchschnittlicher Promillegehalt nach Beendigung der Alkoholaufnahme betrug: 1,04; er durchschnittliche Promillegehalt nach 30 Minuten nach Verabreichung der Zubereitung betrug: 0,52, d.h. es ergab sich eine durchschnittliche Reduktion des Promillegehalts um 0,52
- es konnte kein signifikanter Unterschied in der Wirkung bei Männern und Frauen beobachtet werden
- keine Unverträglichkeiten oder Nebenwirkungen wurden beobachtet
- die subjektive Einschätzung der Reduktion der unmittelbaren Alkoholwirkungen bei den Probanden war positiv.

### Beispiel 3 (Zubereitung ohne Vitamin B Zusatz)

Eine der Zusammensetzung aus Beispiel 1 entsprechende pulverförmige Zubereitung, die jedoch neben der Clinoptilolith-Komponente (mit mittlerer Teilchengröße D-50 von etwa 0,1 mm) keine B-Vitamine enthält, zeigt eine deutlich schwächere Wirkung hinsichtlich der Nebenwirkungen des Alkohols ("hang-over").

## Patentansprüche

1. Zeolith-haltige Zubereitung (Z), die 50 bis 99,99 Gew.-% mindestens einer Clinoptilolith-Komponente (CL) und 50 bis 0,01 Gew.-% mindestens einer weiteren Komponente (K) enthält, wobei die Clinoptilolith-Komponente (CL) durch die Formel (C-1):
(Ca, K₂, Na₂, Mg)₄Al₈Si₄₀O₉₆ x 24 H₂O
beschrieben wird,
wobei die Clinoptilolith-Komponente (CL) fein gemahlen ist und eine mittlere Teilchengröße (D-50) von kleiner als 0,01 mm aufweist, und
wobei eine der weiteren Komponenten (K) eine mit einer Schutzhülle umgebende Carbonat- und/oder Hydrogencarbonat-Komponente ist.

2. Zeolith-haltige Zubereitung (Z) gemäß Patentanspruch 1, **dadurch gekennzeichnet, dass** sie 50 bis 99,99 Gew.-% mindestens einer Clinoptilolith-Komponente (CL) und 50 bis 0,01 Gew.-% an weiteren Komponenten (K) enthält, wobei es sich bei diesen weiteren Komponente (K) einerseits um Hilfskomponenten (H) und andererseits um Vitamin-Komponenten (V) handelt.

3. Zeolith-haltige Zubereitung (Z) gemäß einem der Patentansprüche 1 oder 2, **dadurch gekennzeichnet, dass** sie aus 50 bis 99,99 Gew.-% einer Clinoptilolith-Komponente (CL) und aus 50 bis 0,01 Gew.-% an weiteren Komponenten (K) besteht, wobei eine dieser weiteren Komponente (K) ein Vitamin-B-Komponente ist.

4. Zeolith-haltige Zubereitung (Z) gemäß einem der Patentansprüche 1 bis 3, **dadurch gekennzeichnet, dass** sie aus 50 bis 99,99 Gew.-% einer Clinoptilolith-Komponente (CL) und aus 50 bis 0,01 Gew.-% an weiteren Komponenten (K) besteht, wobei eine dieser weiteren Komponente (K) das Vitamin B12 ist.

5. Zeolith-haltige Zubereitung (Z) gemäß einem der Patentansprüche 1 bis 4, **dadurch gekennzeichnet, dass** sie aus 50 bis 99,99 Gew.-% einer Clinoptilolith-Komponente (CL) und aus 50 bis 0,01 Gew.-% an weiteren Komponenten (K) besteht, wobei eine dieser weiteren Komponente (K) ein Oberflächen-modifizierter Hilfsstoff (H) ist.

6. Zeolith-haltige Zubereitung (Z) gemäß einem der Patentansprüche 1 bis 5, **dadurch gekennzeichnet, dass** es sich um eine orale Zubereitung in Pulverform handelt, die eine Clinoptilolith-Komponente (CL), mehrere Vitamin-B-Komponenten sowie mindestens eine Carbonat- und/oder Hydrogencarbonat-Komponente enthält.

7. Zeolith-haltige Zubereitung (Z) gemäß einem der Patentansprüche 1 bis 6, **dadurch gekennzeichnet, dass** sie enthält:
| | |
|---|---|
| Clinoptilolith | 8 bis 10 Gewichtsteile |
| Vitamine C, D, E, A, B | 0,1 bis 0,5 Gewichtsteile |
| Carbonat-Komponenten | 2 bis 10 Gewichtsteile |
| Citronensäure | 2 bis 5 Gewichtsteile sowie |
| Polymer-Hilfskomponenten | 0 bis 2 Gewichtsteile. |

8. Verfahren zur Herstellung einer Zeolith-haltigen Zubereitung (Z) gemäß einem der Patentansprüche 1 bis 7, bei dem zunächst die Clinoptilolith-Komponente (CL) durch ein Mahlverfahren auf eine mittlere Teilchengröße (D-50) von kleiner als 0,01 mm gebracht wird, und anschließend die Clinoptilolith-Komponente (CL) mit den weiteren Komponenten (K) vermischt wird.

9. Zeolith-haltigen Zubereitung (Z) gemäß einem der Patentansprüche 1 bis 7 zur Verwendung zur Prävention oder Behandlung der Nebenwirkungen von Alkohol.

10. Aufreißbeutel enthaltend 6 bis 15 Gramm einer pulverförmigen, Zeolith-haltigen Zubereitung (Z) gemäß einem der Patentansprüche 1 bis 7.

11. Aufreißbeutel enthaltend 6 bis 15 Gramm einer pulverförmigen, Zeolith-haltigen Zubereitung (Z) nach Anspruch 10, **dadurch gekennzeichnet, dass** sie enthält:
| | |
|---|---|
| Clinoptilolith | 8 bis 10 Gewichtsteile |
| Vitamine C, D, E, A, B | 0,1 bis 0,5 Gewichtsteile |
| Carbonat-Komponenten | 2 bis 10 Gewichtsteile |
| Citronensäure | 2 bis 5 Gewichtsteile sowie |
| Polymer-Hilfskomponenten | 0 bis 2 Gewichtsteile. |

## Claims

1. Zeolite-containing preparation (Z), which contains from 50 to 99.99% by weight of at least one clinoptilolite component (CL) and from 50 to 0.01% by weight of at least one further component (K), wherein the clinoptilolite component (CL) is described by the formula (C-1):
(Ca, K₂, Na₂, Mg)₄Al₈Si₄₀O₉₆ × 24 H₂O
and the clinoptilolite component (CL) is finely milled and has an average particle size (D-50) of less than 0.01 mm and
one of the further components (K) is a carbonate and/or hydrogencarbonate component surrounded by a protective shell.

2. Zeolite-containing preparation (Z) according to Claim 1, **characterized in that** it contains from 50 to 99.99% by weight of at least one clinoptilolite component (CL) and from 50 to 0.01% by weight of further components (K), where these further components (K) are firstly auxiliary components (H) and secondly vitamin components (V).

3. Zeolite-containing preparation (Z) according to either Claim 1 or 2, **characterized in that** it consists of from 50 to 99.99% by weight of a clinoptilolite component (CL) and from 50 to 0.01% by weight of further components (K), where one of these further components (K) is a vitamin B component.

4. Zeolite-containing preparation (Z) according to any of Claims 1 to 3, **characterized in that** it consists of from 50 to 99.99% by weight of a clinoptilolite component (CL) and from 50 to 0.01% by weight of further components (K), where one of these further components (K) is vitamin B12.

5. Zeolite-containing preparation (Z) according to any of Claims 1 to 4, **characterized in that** it consists of from 50 to 99.99% by weight of a clinoptilolite component (CL) and from 50 to 0.01% by weight of further components (K), where one of these further components (K) is a surface-modified auxiliary (H).

6. Zeolite-containing preparation (Z) according to any of Claims 1 to 5, **characterized in that** it is an oral preparation in powder form which contains a clinoptilolite component (CL), a plurality of vitamin B components and at least one carbonate and/or hydrogencarbonate component.

7. Zeolite-containing preparation (Z) according to any of Claims 1 to 6, **characterized in that** it contains:
| | |
|---|---|
| clinoptilolite | from 8 to 10 parts by weight |
| vitamins C, D, E, A, B | from 0.1 to 0.5 part by weight |
| carbonate components | from 2 to 10 parts by weight |
| citric acid | from 2 to 5 parts by weight and |
| polymer auxiliary components | from 0 to 2 parts by weight. |

8. Process for producing a zeolite-containing preparation (Z) according to any of Claims 1 to 7, wherein the clinoptilolite component (CL) is firstly brought to an average particle size (D-50) of less than 0.01 mm by a milling process and the clinoptilolite component (CL) is subsequently mixed with the further components (K).

9. Zeolite-containing preparation (Z) according to any of Claims 1 to 7 for use for the prevention or treatment of the secondary effects of alcohol.

10. Tear-open sachet containing from 6 to 15 gram of a pulverulent, zeolite-containing preparation (Z) according to any of Claims 1 to 7.

11. Tear-open sachet containing from 6 to 15 gram of a pulverulent, zeolite-containing preparation (Z) according to Claim 10, **characterized in that** it contains:
| | |
|---|---|
| clinoptilolite | from 8 to 10 parts by weight |
| vitamins C, D, E, A, B | from 0.1 to 0.5 part by weight |
| carbonate components | from 2 to 10 parts by weight |
| citric acid | from 2 to 5 parts by weight and |
| polymer auxiliary components | from 0 to 2 parts by weight. |

## Revendications

1. Préparation contenant une zéolithe (Z), qui contient 50 à 99,99 % en poids d'au moins un composant clinoptilolithe (CL) et 50 à 0,01 % en poids d'au moins un autre composant (K), le composant clinoptilolithe (CL) étant décrit par la formule (C-1) :
(Ca, K₂, Na₂, Mg)₄Al₈Si₄₀O₉₆ x 24 H₂O
le composant clinoptilolithe (CL) étant finement broyé et présentant une taille de particule moyenne (D-50) de moins de 0,01 mm, et
un des autres composants (K) étant un composant carbonate et/ou hydrogénocarbonate entouré par une enveloppe de protection.

2. Préparation contenant une zéolithe (Z) selon la revendication 1, **caractérisée en ce qu'**elle contient 50 à 99,99 % en poids d'au moins un composant clinoptilolithe (CL) et 50 à 0,01 % en poids d'autres composants (K), ces autres composants (K) consistant d'une part en des composants auxiliaires (H) et d'autre part en des composants vitamines (V).

3. Préparation contenant une zéolithe (Z) selon l'une quelconque des revendications 1 ou 2, **caractérisée en ce qu'**elle contient 50 à 99,99 % en poids d'un composant clinoptilolithe (CL) et 50 à 0,01 % en poids d'autres composants (K), un de ces autres composants (K) étant un composant vitamine B.

4. Préparation contenant une zéolithe (Z) selon l'une quelconque des revendications 1 à 3, **caractérisée en ce qu'**elle contient 50 à 99,99 % en poids d'un composant clinoptilolithe (CL) et 50 à 0,01 % en poids d'autres composants (K), un de ces autres composants (K) étant la vitamine B12.

5. Préparation contenant une zéolithe (Z) selon l'une quelconque des revendications 1 à 4, **caractérisée en ce qu'**elle contient 50 à 99,99 % en poids d'un composant clinoptilolithe (CL) et 50 à 0,01 % en poids d'autres composants (K), un de ces autres composants (K) étant un adjuvant à surface modifiée (H).

6. Préparation contenant une zéolithe (Z) selon l'une quelconque des revendications 1 à 5, **caractérisée en ce qu'**il s'agit d'une préparation orale sous la forme de poudre, qui contient un composant clinoptilolithe (CL), plusieurs composants vitamines B, ainsi qu'au moins un composant carbonate et/ou hydrogénocarbonate.

7. Préparation contenant une zéolithe (Z) selon l'une quelconque des revendications 1 à 6, **caractérisée en ce qu'**elle contient :
| | |
|---|---|
| clinoptilolithe | 8 à 10 parties en poids, |
| vitamines C, D, E, A, B | 0,1 à 0,5 partie en poids, |
| composants carbonate | 2 à 10 parties en poids, |
| acide citrique | 2 à 5 parties en poids, et |
| composants adjuvants polymères | 0 à 2 parties en poids. |

8. Procédé de fabrication d'une préparation contenant une zéolithe (Z) selon l'une quelconque des revendications 1 à 7, selon lequel le composant clinoptilolithe (CL) est tout d'abord amené par un procédé de broyage à une taille de particule moyenne (D-50) de moins de 0,01 mm, puis le composant clinoptilolithe (CL) est mélangé avec les autres composants (K).

9. Préparation contenant une zéolithe (Z) selon l'une quelconque des revendications 1 à 7, destinée à une utilisation pour la prévention ou le traitement des effets secondaires de l'alcool.

10. Sachet à ouverture déchirable contenant 6 à 15 grammes d'une préparation en forme de poudre contenant une zéolithe (Z) selon l'une quelconque des revendications 1 à 7.

11. Sachet à ouverture déchirable contenant 6 à 15 grammes d'une préparation en forme de poudre contenant une zéolithe (Z) selon la revendication 10, **caractérisé en ce qu'**il contient :
| | |
|---|---|
| clinoptilolithe | 8 à 10 parties en poids, |
| vitamines C, D, E, A, B | 0,1 à 0,5 partie en poids, |
| composants carbonate | 2 à 10 parties en poids, |
| acide citrique | 2 à 5 parties en poids, et |
| composants adjuvants polymères | 0 à 2 parties en poids. |
